# EUROPEAN PATENT SPECIFICATION

(11) **EP 1 049 419 B1**
(45) Date of publication and mention of the grant of the patent: **10.08.2005**
(21) Application number: 99900719.8
(22) Date of filing: 20.01.1999
(51) Int. Cl.: A61F 2/06, F16L 11/12, B29C 35/08

(54) **A METHOD FOR MAKING AN EXTENSIBLE STENT FROM SHEETMATERIAL**
VERFAHREN ZUR HERSTELLUNG EINES AUS BANDFÖRMIGEN MATERIAL, AUSDEHNBAREN STENTS
PROCEDE DE FABRICATION D'UNE ENDOPROTHESE EXTENSIBLE A PARTIR D'UNE MATIERE DE FEUILLE

(30) Priority: 22.01.1998 NL 1008093
(43) Date of publication of application: 08.11.2000
(73) Proprietor: Rigitec B.V., 7537 PG Enschede (NL)
(72) Inventor: GLASTRA, Hendrik, NL-7537 PG Enschede (NL)
(74) Representative: Timmers, Cornelis Herman Johannes
(86) International application number: PCT/NL1999/000035
(87) International publication number: WO 1999/037243

(56) References cited:
- EP-A- 0 689 807
- EP-A- 0 695 626
- EP-A- 0 737 453
- EP-A- 0 815 760
- FR-A- 2 694 688
- GB-A- 2 115 776
- GB-A- 2 270 264
- US-A- 4 997 703
- US-A- 5 397 365

## Description

The invention relates to a method for making an extensible stent which is ultimately brought into a form retaining state, using absorbent material soaked with a plastics the curing of which can be initiated controllably.

Such a vascular stent for locally reinforcing a body vessel, is introduced by means of a catheter and thereafter brought into its final configuration by means of an extensible element at the distal end of this catheter, whereafter irradiation with UV radiation initiates the curing of the plastics material. Such a stent is known from the prior art, particularly EP-0 617 930, having the same inventor as the present one. In this known stent the absorbent material is enclosed in a thin-walled sleeve, closed at both ends.

The fabrication of such a stent is cumbersome and costly, the more so because a great number of stents with many different lengths and diameters for coping with different situations and circumstances must be fabricated and kept in store.

Despite the ongoing development in the field of stents there is as yet no method by means of which stents can be made quickly and cheaply even shortly before the introduction thereof into a body vessel, this in every conceivable desired final configuration while meeting all requirements.

The invention aims to provide such a method.

To this end the invention proposes a method according to which said material is sheet-shaped with coherent fibres and is wound around a template having an outer configuration corresponding with the desired final configuration to obtain a semi-finished article with stable shape.

This plastics can be dissolved in a solvent which thereafter is evaporated controllable by heating.

Making a template, which, after winding it in the way as proposed by the invention results into a stent which is completely suited to a specific situation, particularly a situation which is evaluated on the hand of X-ray images of a body vessel which is to be treated, can be done cheaply. It is possible, after making the template, to compare the dimensions and configurations thereof with X-ray images of the body vessel in question, whereafter, if possible, configuration and dimensions of the template can be corrected. Thereafter, the stent is made in the way as proposed by the invention and this stent is then fit to be introduced into the body vessel. If necessary the making of the final stent can be preceded by making a trial model.

Preferred embodiments of the method according to the invention are described in the claims 2 up to and including 7.

The fact that the method according to the invention is excellently suited to provide thereon a therapeutic active for a radiation emitting substance leads to the advantage that it is possible to implant it into a patient directly after the determination of shape and configuration of such a stent, so that the treatment can be started without any delay.

In a preferred embodiment of the method according to the invention one will use a sheet-shaped base material with metallic reinforcing fibers distributed therein. With these simple measures which can be integrated simply in the fabrication process of the base material an increased strength of the material is obtained so that for a particular use it can have smaller dimensions, particularly a smaller thickness, in combination with a certain opaqueness for X-ray radiation so that not only the positioning but also the detection of a stent made from this material at a later moment becomes very easy.

Length, diameter and configuration of the fibers will, in a certain practical embodiment, be adapted to the use one has in view. Preferred embodiments are given in the claims 9 and 10.

Further preferred embodiments for using this material are described in the claims 11 up to and including 16.

The measure according to claim 17 gives the possiblity for a local treatment with radio-active radiation.

The starting material for the fibers can be made radio-active by introducing a radio-active isotope in the melt thereof. Of course then when handling and using the material the necessary safety measures must be taken but as the intensitivity of the radiation is only low - this as a result of the short distance between stent and tissue to be treated - this will not result into any insurmountable problems in practice.

The claimed exclusive rights also include a stent such as is subject of claim 19.

The invention is elucidated on the hand of a drawing. Therein show:
fig. 1 schematically the use of a template in the method according to the invention;
fig. 2 in cross-section a preferred embodiment of a stent, obtained therewith;
fig. 3 the use of a template to execute the method according to the invention in combination with the provision of openings in the surface of the stent obtained therewith;
fig. 4 a side view of a stent, obtained in this way;
fig. 5 a side view of a template and the stent formed thereon, having a tapering configuration;
fig. 6 a side view of a template with a stent made thereon in a configuration in which the ends of the stent have a greater diameter than the middle part thereof;
fig. 7 schematically and in top view sheet-shaped material to be used in the method according to the invention;
fig. 8 schematically and in perspective a stent made from this material;
fig. 9 schematically and in top view sheet-shaped material to be used in the method according to the invention according to a second embodiment;
fig. 10 schematically and in perspective view another embodiment of a stent obtained by using this material;
fig. 12 in top view a third embodiment of sheet-shaped starting material;
fig. 13 a fourth embodiment of the sheet-shaped starting material according to the invention; and
fig. 15 in perspective view a stent made therefrom.

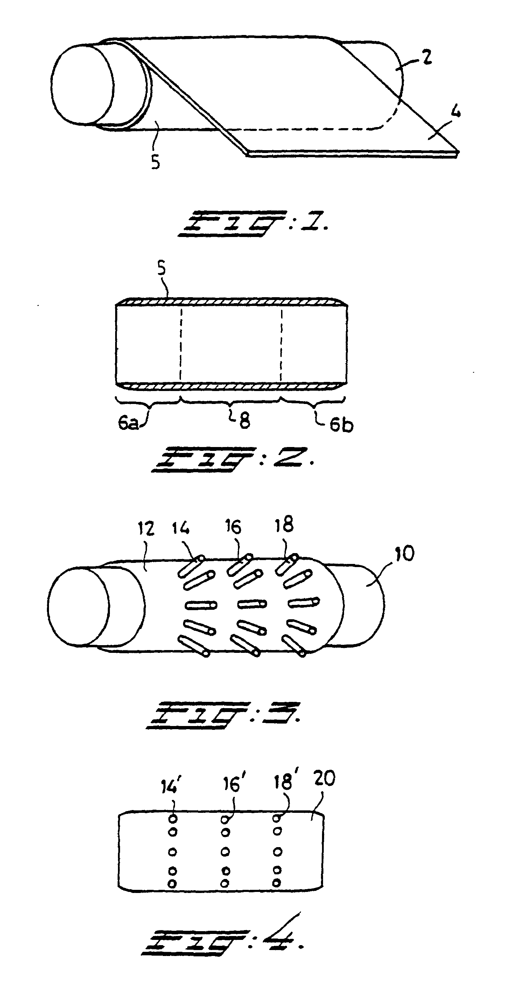

The method according to the invention will be elucidated on the hand of the schematic fig 1. On a template 2 with in this example, a cylindrical outer surface a strip of flexible material 4 is wound which is made from mutually coherent fibers; a good starting material for executing the method according to the invention is thin and pure paper. It is clear that the drawing of fig. 1 is not on scale; this also goes for the other drawings to be discussed hereinafter.

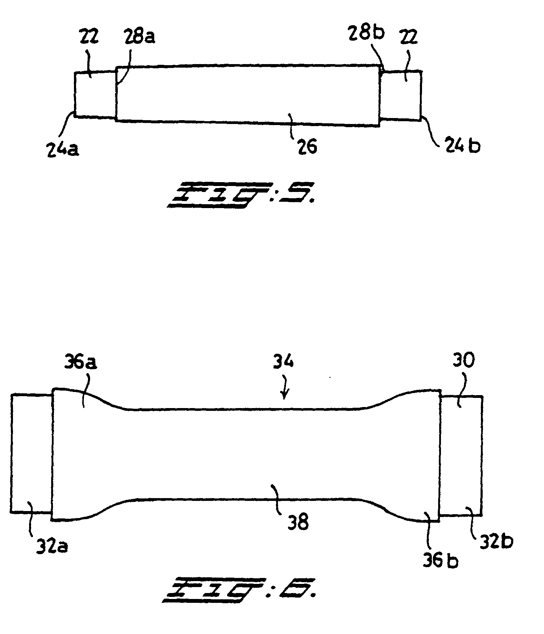

After winding such a length of material on the template 2 that the wound cylinder 5 has obtained the desired thickness the material is cut-off and thereafter soaked in a plastics, which can be dissolved in a solvent of the kind known as such, the curing of the plastics can be initiated controllably by irradiation with UV-radiation. One can also use a fiber material which is soaked in such a plastics before it is wound, but handling thereof is somewhat more difficult. After removal of the template 2 and, if necessary, heating in an oven, one obtains a stent 5 with the cylindrical configuration as shown in fig. 2 in cross-section. Thereafter this stent, of which the outer surface is still somewhat sticky, can be rolled in a therapeutic substance (aspirin, heparin, cocain) in powder form, which adheres to the outer surface and can easily be provided thereon in a controlled quantity. Controlled powdering is also possible.

For many uses it is desired that the ends of a stent are more flexible than the middle part and according to the invention this can be obtained very simply by chosing a higher concentration of the plastics material in the end zones, indicated in fig. 2 with 6a and 6b, than in the middle zone 8.

In some cases it is desired that a substance with a therapeutic action, or a substance emitting radio-active radiation is provided not over the entire surface of the stent but only in a certain part thereof. The method of the invention makes it easy to coat the, still sticky, outer surface of the stent with a substance with therapeutic action, not equally, but only selectively on certain surface parts. The invention also gives the possibility to form openings on certain preferred places in the stent, said openings being filled with a substance with therapeutic action later-on.

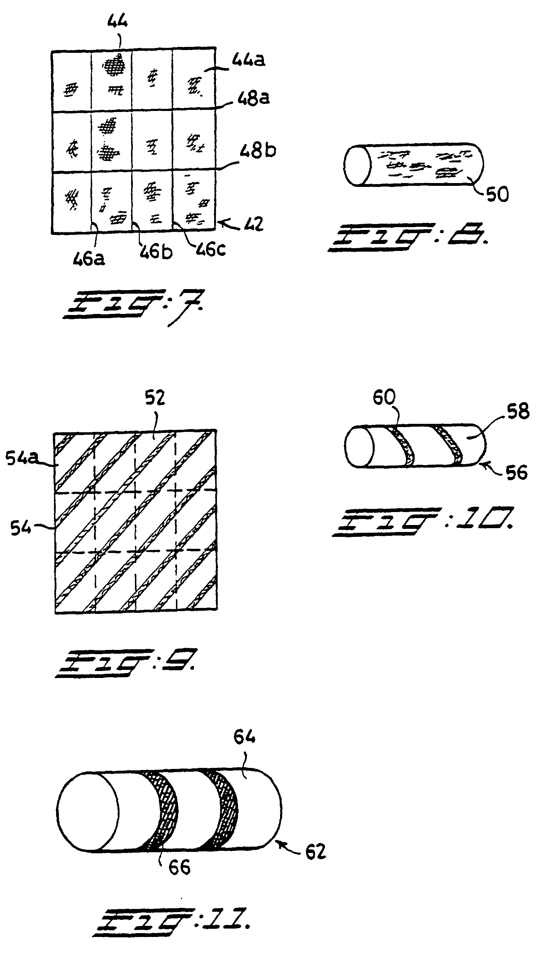

To this end one can, as fig. 3 shows, use a template 10 in which the stent 12, wound thereon, is pierced by pins, placed in rings, as indicated by 14, 16 en 18. After curing of the stent and the removal of the pins the stent 20 results within its body three rings of openings 14', 16', 18', corresponding with the rings of pins 14, 16 and 18.

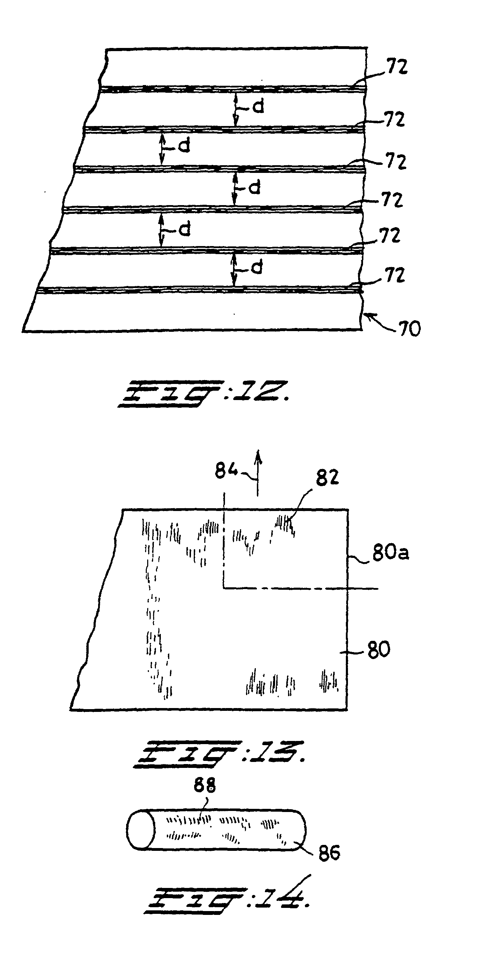

One is, of course, totally free in chosing the shape of the stent as this is completely determined by the outer shape of the template. Fig. 5 shows a somewhat tapering template 22 of which the left hand end 24a has a smaller diameter than the right hand end 24b; corresponding therewith the stent 26, obtained with this template, is also tapering; the left hand end 28a has a smaller diameter than the right hand end 28b. This stent can be removed easily from the template 22.

Making a stent of which the outer ends have a greater diameter than the middle part poses no problem. Fig. 6 shows in side view such an embodiment of the method according to the invention: the template 30, as used, has ends 32a, 32b with a greater diameter than the middle part and corresponding therewith the stent 34, formed thereon, has ends 36a, 36b with a greater diameter than the middle part 38.

Of course one will use in this case a template with two aligned parts which are removed to the left and right respectively from the stent 38.

According to a advantageous embodiment of the invention it is proposed to introduce and distribute metallic reinforcing fibers into the sheet-shaped starting material. These fibers are preferably made from titanium, generally from a "biocompatible" material. This introducing can be done during the grinding of the fibers or also during the intensive mixing of the pulp.

The length and the diameter of the fibers to be added can be chosen in dependance on the final use one has in mind. The fibers can be supplied individually and incoherently but it is also possible to introduce them more or less coherently into the pulp, as will be described later on.

As a value for the minimum diameter to be used one thinks of 5 µm and as minimum length 1-2 mm, although the dimensions are certainly not restrictive and particularly the minimum dimensions are only set by the possibility of handling the fibers. The weight percentage of the fibers can be chosen independant from the aim one has in mind: a weight percentage of 10-20% is possible. Other values are also possible.

It is, of course, possible to mix the fibers with the plastics material before the sheet material is soaked with it.

The introduced metallic fibers are of course impervious to UV radiation, which could result in the problem that the curing of the plastics, being started by irradation with UV radiation will not proceed evenly in a stent made from the material according to the invention. In order to ensure that in any case an important part of the stent, made from the material according to the invention is directly irradiated with UV radiation it is proposed not to introduce the fibers equally into the starting material but according to a certain stripe pattern.

Fig. 7 shows a part of sheet material 42 with equally distributed metallic fibers 44 therein, which are, of course, shown only schematically. The sheet 42 is cut up along the perpendicular crossing vertical cutting lines 46a, 46b, 46c to individual sheets 4a, from which the stent 50, shown in perspective view in fig. 8, is made. Preferably however, the fibers are not distributed equally but according to a pattern of mutual parallel lines which include an angle with the boundaries of the sheet 44, such as shown in fig. 9, according to which the sheet 42 is provided with a pattern of strips 54 in which metallic fibers are embedded in the starting material. So a stent as shown in fig. 10 and indicated therein with reference numeral 56, made from such a sheet 54, has strips 60 which run obliquely over the outer surface 58 and in which the metallic fibers, which give the stent 56 its strength, are embedded in the material. In order to initiate the curing the sleeve is irradiated from within with UV radiation and practically the whole inner surface will then be irradiated equally.

When the fibers are prepared in such a way that they emit radio-active radiation the stent also has a therapeutic action.

According to the invention it is furthermore possible to wind a stent in the way as shown schematically and in perspective view in fig. 11: a sleeve 62 from normal fiber material which has none or only a very small percentage of metallic fibers supports, on the outer surface 64 thereof, a helically wound strip 66 which is made of sheet material, soaked with curing plastics and having therein a certain concentration metallic fibers. Here, too, the irradiation of the curing plastics will take place starting from the center of the core so that the base layer 64 will cure swiftly and uniformly; however after this layer 24 sufficient UV radiation reaches the strip 26 to initiate the curing thereof. This embodiment has the advantage that one can choose the composition of the plastics present in the strips 66 in such a way that the curing of the basic core 64 and the strips 66 occurs equally fast.

The aspect discussed above according to which the fibers are distributed strip-like in the basic material can also be realised in the way as shown in fig. 12. The fibers will be made generally by drawing wire-shaped starting material and in the known manner they are then available in bundles; from such a bundle of fibers smaller bundles are made which are embedded with predetermined mutual distances in the pulp material. Fig. 12 shows a part of a pulp bed indicated with reference numeral 90; with predetermined mutual distances d fiber bundles 92 are embedded therein. Finally one obtains a starting material which is reinforced in a strip-like manner and from which stents having the configuration according to fig. 4 can be made.

Fig. 13 shows a fiber material 80 having therein elongate fibers 82 which are all directed in the same direction indicated by the arrow 84. When a part 80a is separated therefrom, from which part finally a cylindrical stent 86 according to fig. 8 is made, all fibers 88 therein will be oriented in the circumferential direction. In this way this element has an increased radial strength.

After making the stent as described hereabove this stent can be coated with the commercially available "chronoflex"®, whereafter the whole is baked at a suitable temperature. This results in a biocompatible outer surface so that the material can be used without problems in a body vessel.

## Claims

1. Method for making an extensible stent which is ultimately brought into a form retaining state, using absorbent material soaked with a plastics the curing of which can be initiated controllably, **characterized in that** this material is sheet-shaped with coherent fibres and is wound around a template having an outer configuration corresponding with the desired final configuration to obtain a semi-finished article with stable shape.

2. Method according to claim 1, **characterised in that** one supplies the plastics dissolved in a solvent and removes this solvent by heating the soaked sheet-material.

3. Method according to claims 1-2 in which one provides on the still sticky, outer surface of the stent, either by powdering or by rolling, a therapeutically active or radio-active radiation emitting substance.

4. Method according to claims 1-3 in which one provides in the surface holes destined to be filled with a substance which is therapeutically active or emits radio-active radiation.

5. Method according to claim 4,'in which one forms the holes by piercing the wound material with pins with a configuration corresponding to the desired holes.

6. Method according to claims 1-5, in which one distributes the plastics inhomogenously over the stent.

7. Method according to claim 6, in which one choses a smaller concentration of the plastics in the fiber material at the ends of the stent than in the middle part thereof.

8. Method according to claims 1-7, in which one uses a sheet-shaped starting material with metallic reinforcing fibers distributed therein.

9. Method according to claim 8, in which the diameter of the reinforcing fibers is chosen to lie between 2 µm and 500 µ.

10. Method according to claims 8-9, in which the length of the reinforcing fibers is chosen to lie between 2 mm and 10 mm.

11. Method according to claims 8-9, in which one uses elongate reinforcing fibers.

12. Method according to claims 8-10, in which one uses curved reinforcing fibers.

13. Method according to claims 8-12, in which one uses titanium reinforcing fibers.

14. Method according to claims 8-13, in which one concentrates the reinforcing fibers according to a predetermined stripe pattern.

15. Method according to claim 14, in which one brings the reinforcing fibers in bundles in the base-layer.

16. Method according to claims 8-15, in which one uses elongate, essentially equally directed, fibers.

17. Method according to claims 8-12, in which one uses fibers prepared to emit radio-active radiation.

18. Method according to claims 8-17, in which the obtained stent is coated with a coating of chronoflex®.

19. Stent obtainable by using the method according to one or more of the claims 1-18, **characterised in that** the diameter at the outer ends (36a, 36b) thereof is greater than same of the middle part (38).

## Patentansprüche

1. Verfahren zur Herstellung eines dehnbaren Stents, der zum Schluss in einen formbeständigen Zustand überführt wird, wobei ein mit Kunststoff getränktes absorbierendes Material verwendet wird, dessen Aushärtung steuerbar einzuleiten ist, **dadurch gekennzeichnet, dass** das genannte Material eine Blattform mit kohärenten Fasern besitzt und um eine Formvorlage gewickelt wird, die eine äußere Konfiguration hat, die der gewünschten endgültigen Konfiguration entspricht, um ein Halbfertigprodukt mit stabiler Gestalt zu erhalten.

2. Verfahren nach Anspruch 1, **dadurch gekennzeichnet, dass** man den Kunststoff in einem Lösungsmittel gelöst zuführt und das Lösungsmittel durch Erwärmen des getränkten bandförmigen Materials wieder entfernt.

3. Verfahren nach den Anspruch 1 - 2, **dadurch gekennzeichnet, dass** man auf die noch klebrige Außenseite des Stents eine therapeutisch wirksame oder eine radioaktive Strahlung emittierende Substanz durch Pudern oder Aufrollen aufträgt.

4. Verfahren nach Anspruch 1 - 3, **dadurch gekennzeichnet, dass** man in der Oberfläche Löcher vorsieht, die dazu bestimmt sind, mit einer therapeutisch wirksame oder radioaktive Strahlung abgebenden Substanz gefüllt zu werden.

5. Verfahren nach Anspruch 4, **dadurch gekennzeichnet, dass** die Löcher durch Perforation des gewickelten Materials mit Stiften erzeugt werden, die der Konfiguration der gewünschten Löcher entsprechen.

6. Verfahren nach Anspruch 1 - 5, **dadurch gekennzeichnet, dass** der Kunststoff ungleichmäßig über den Stent verteilt wird.

7. Verfahren nach Anspruch 6, **dadurch gekennzeichnet, dass** am Ende des Stents eine geringere Konzentration des Kunststoffs in dem Fasermaterial gewählt wird als im mittleren Bereich.

8. Verfahren nach Anspruch 1 - 7, **dadurch gekennzeichnet, dass** man ein blattförmiges Ausgangsmaterial mit darin verteilten metallischen Verstärkungsfasern benutzt.

9. Verfahren nach Anspruch 8, **dadurch gekennzeichnet, dass** der Durchmesser der Verstärkungsfasern zwischen 2 µm und 500 µm gewählt wird.

10. Verfahren nach Anspruch 8 - 9, **dadurch gekennzeichnet, dass** die Länge der Verstärkungsfasern zwischen 2 mm und 10 mm gewählt wird.

11. Verfahren nach Anspruch 8 - 9, **dadurch gekennzeichnet, dass** gelängte Verstärkungsfasern gewählt werden.

12. Verfahren nach Anspruch 8 - 10, **dadurch gekennzeichnet, dass** gekrümmte Verstärkungsfasern verwendet werden.

13. Verfahren nach Anspruch 8 - 12, **dadurch gekennzeichnet, dass** Verstärkungsfasern aus Titan benutzt werden.

14. Verfahren nach Anspruch 8 - 13, **dadurch gekennzeichnet, dass** die Verstärkungsfasern gemäß einem vorher festgelegten Streifenmuster zusammengefasst werden.

15. Verfahren nach Anspruch 14, **dadurch gekennzeichnet, dass** die Verstärkungsfasern in Bündeln in die BasisSchicht eingebracht werden.

16. Verfahren nach Anspruch 8 - 15, **dadurch gekennzeichnet, dass** gedehnte und im Wesentlichen gerichtete Fasern benutzt werden.

17. Verfahren nach Anspruch 8 - 12, **dadurch gekennzeichnet, dass** Fasern benutzt werden, die so präpariert sind, dass sie radioaktive Strahlung emittieren.

18. Verfahren nach Anspruch 8 - 17, **dadurch gekennzeichnet, dass** der erhaltene Stent mit einer Schicht aus Chronoflex® versehen ist.

19. Nach dem Verfahren gemäß einem oder mehreren der Ansprüche 1 - 18 herzustellender Stent, **dadurch gekennzeichnet, dass** der Durchmesser an den äußeren Enden (3ba, 3bb) größer ist als der Durchmesser im mittleren Teil (38) des Stents.

## Revendications

1. Procédé de fabrication d'une endoprothèse extensible qui est finalement amenée dans un état où elle conserve sa forme, en utilisant un matériau absorbant trempé dans un plastique dont le durcissement peut être lancé de manière contrôlée, **caractérisé en ce que** ce matériau est façonné en feuille avec des fibres cohérentes et est enroulé autour d'un gabarit qui possède une configuration extérieure correspondant à la configuration finale désirée pour obtenir un article semi-fini ayant une forme stable.

2. Procédé selon la revendication 1, **caractérisé en ce que** l'on fournit le plastique dissout dans un solvant et **en ce que** l'on enlève ce solvant en chauffant le matériau trempé façonné en forme de feuille.

3. Procédé selon les revendications 1 et 2, dans lequel on fournit sur la surface extérieure encore collante de l'endoprothèse, soit par farinage, soit par laminage, une substance thérapeutiquement active ou émettant un rayonnement radioactif.

4. Procédé selon les revendications 1 à 3, dans lequel on fournit sur la surface des trous destinés à être remplis d'une substance qui est thérapeutiquement active ou qui émet un rayonnement radioactif.

5. Procédé selon la revendication 4, dans lequel on forme les trous en perçant le matériau enroulé avec des tiges présentant une configuration correspondant aux trous souhaités.

6. Procédé selon les revendications 1 à 5, dans lequel on répartit le plastique de manière non homogène sur l'endoprothèse.

7. Procédé selon la revendication 6, dans lequel on choisit une concentration de plastique dans le matériau fibreux plus faible aux extrémités de l'endoprothèse que dans le milieu de celle-ci.

8. Procédé selon les revendications 1 à 7, dans lequel on utilise un matériau de départ façonné en feuille avec des fibres métalliques de renforcement réparties à l'intérieur de celui-ci.

9. Procédé selon la revendication 8, dans lequel le diamètre des fibres de renforcement est choisi pour être compris entre 2 µm et 500 µ.

10. Procédé selon les revendications 8 à 9, dans lequel la longueur des fibres de renforcement est choisie pour être comprise entre 2 mm et 10 mm.

11. Procédé selon les revendications 8 à 9, dans lequel on utilise des fibres de renforcement étirées.

12. Procédé selon les revendications 8 à 10, dans lequel on utilise des fibres de renforcement courbées.

13. Procédé selon les revendications 8 à 12, dans lequel on utilise des fibres de renforcement en titane.

14. Procédé selon les revendications 8 à 13, dans lequel on concentre les fibres de renforcement selon un motif strié prédéterminé.

15. Procédé selon la revendication 14, dans lequel on amène les fibres de renforcement en bottes dans la couche de base.

16. Procédé selon les revendications 8 à 15, dans lequel on utilise des fibres étirées, orientées sensiblement dans la même direction.

17. Procédé selon les revendications 8 à 12, dans lequel on utilise des fibres préparées pour émettre un rayonnement radioactif.

18. Procédé selon les revendications 8 à 17, dans lequel l'endoprothèse obtenue est revêtue d'un revêtement de Chronoflex®.

19. Endoprothèse pouvant être obtenue en utilisant le procédé selon l'une ou plusieurs des revendications 1 à 18, **caractérisée en ce que** le diamètre aux extrémités extérieures (36a, 36b) de celle-ci est plus grand que celui de la partie du milieu (38).
